Europäisches Patentamt
European Patent Office
Office européen des brevets

(19)

(11) Publication number: **0 127 245**
**A2**

(12)

# EUROPEAN PATENT APPLICATION

(21) Application number: 84200786.6

(22) Date of filing: 30.05.84

(51) Int. Cl.³: C 07 D 233/61
C 07 D 235/06, C 07 D 235/08
C 07 D 249/08, C 07 D 231/16
C 07 D 231/12, C 07 D 403/12
C 07 C 157/14, A 01 N 47/42
A 01 N 47/44

(30) Priority: 31.05.83 JP 94839/83
21.07.83 JP 131989/83
04.10.83 JP 185525/83

(43) Date of publication of application:
05.12.84 Bulletin 84/49

(84) Designated Contracting States:
AT BE CH DE FR GB IT LI NL

(71) Applicant: NIPPON SODA CO., LTD.
2-1, Ohte-machi 2-chome
Chiyoda-ku, Tokyo, 100(JP)

(72) Inventor: Ikura, Katsuyata
2-16-24, Fujimigaoka Ninomiyamachi
Naka-gun Kanagawa-ken(JP)

(72) Inventor: Hayakawa, Koichi
1133-1 Kokufu Hongou Ohiso-machi
Naka-gun Kanagawa-ken(JP)

(72) Inventor: Yamada, Tomio
28-3 Kurobeoka
Hiratsuka-shi Kanagawa-ken(JP)

(72) Inventor: Takahashi, Hidemitsu
420-2 Nagamochi
Hiratsuka-shi Kanagawa-ken(JP)

(72) Inventor: Hatano, Renpei
2-6-23 Koma Ohiso-machi
Naka-gun Kanagawa-ken(JP)

(74) Representative: van der Beek, George Frans et al,
Nederlandsch Octrooibureau Johan de Wittlaan 15 P.O. Box 29720
NL-2502 LS The Haque(NL)

(54) **Carboxyamidine derivatives.**

(57) A compound having the formula

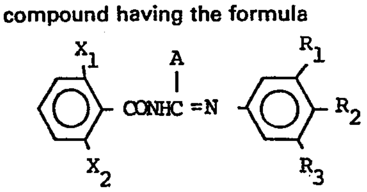

wherein each of $X_1$ and $X_2$ is hydrogen, halogen, methyl, trifluormethyl, with the proviso that $X_1$ and $X_2$ can not be hydrogen at the same time, and

each of $R_1$ and $R_3$ is hydrogen, halogen, nitro, $C_{1-6}$ haloalkyl, $C_{2-6}$ alkenyloxy, or aryloxy radical selected from a group consisting of phenoxy and quinoxalinyloxy which may be substituted by nitro and/or $C_{1-6}$ haloalkyl on said aromatic ring; and $R_2$ is hydrogen, halogen, nitro, $C_{1-8}$ alkyl, $C_{1-3}$ haloalkyl, $C_{1-6}$ alkoxycarbonyl, dialkylamino, phenylazo which may be substituted by $C_{1-3}$ alkyl, or Y-$R_4$, wherein Y is O or S and $R_4$ is $C_{1-6}$ alkyl, $C_{1-6}$ haloalkyl, $C_{2-6}$ alkenyl, $C_{2-6}$ haloalkenyl, $C_{2-6}$ alkynyl, phenylalkyl which may be substituted by halogen and/or $C_{1-3}$ haloalkyl, aromatic radical selected from a group consisting of phenyl, pyridyl and quinoxalinyl which may be substituted by halogen, nitro, cyano and/or $C_{1-3}$ haloalkyl on said aromatic ring; and

A is Az or –SS–$R_5$, wherein Az is heterocyclic radical selected from a group consisting of 1-benzimidazolyl, 1-benztriazolyl and 1-pyrazolyl, which may be substituted by $C_{1-3}$ alkyl and/or halogen, or heterocyclic radical selected from a group consisting of 1-triazolyl and 1-imidazolyl, which may be substituted by $C_{1-3}$ alkyl, and $R_5$ is $C_{1-18}$ alkyl, $C_{3-8}$ cycloalkyl, phenylalkyl of phenyl which may be substituted by halogen, nitro and/or $C_{1-8}$ alkyl; or its metal salt; and

an insecticide containing said compound and/or salt.

Croydon Printing Company Ltd.

# SPECIFICATION

## Carboxyamidine Derivatives

The present invention relates to carboxyamidine derivatives, insecticidal compositions in the form of mixture of such compounds with inert carriers, and a process for the preparation of such compounds.

According to a first aspect of the present invention, there is provided a compound having the formula

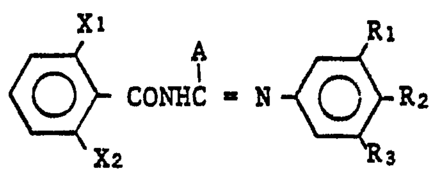

[I]

wherein each of $X_1$ and $X_2$ is hydrogen, halogen, methyl, trifluormethyl, with

the proviso that $X_1$ and $X_2$ can not be hydrogen at the same time, and each of $R_1$ and $R_3$ is hydrogen, halogen, nitro, $C_{1-6}$ haloalkyl, $C_{2-6}$ alkenyloxy, or aryloxy radical selected from a group consisting of phenoxy and quinoxalinyloxy which may be substituted by nitro and/or $C_{1-6}$ haloalkyl on said aromatic ring; and

$R_2$ is hydrogen, halogen, nitro, $C_{1-8}$ alkyl, $C_{1-3}$ haloalkyl, $C_{1-6}$ alkoxycarbonyl, dialkylamino, phenylazo which may be substituted by $C_{1-3}$ alkyl, or $Y-R_4$, wherein Y is O or S and $R_4$ is $C_{1-6}$ alkyl, $C_{1-6}$ haloalkyl, $C_{2-6}$ alkenyl, $C_{2-6}$ haloalkenyl, $C_{2-6}$ alkynyl, phenylalkyl which may be substituted by halogen and/or $C_{1-3}$ haloalkyl, aromatic radical selected from a group consisting of phenyl, pyridyl and quinoxalinyl which may be substituted by halogen, nitro, cyano and/or $C_{1-3}$ haloalkyl on said aromatic ring; and

A is Az or $-SS-R_5$, wherein Az is heterocyclic radical selected from a group consisting of 1-benzimidazolyl, 1-benztriazolyl and 1-pyrazolyl, which may be substituted by $C_{1-3}$ alkyl and/or halogen, or heterocyclic radical selected from a group consisting of 1-triazolyl and 1-imidazolyl, which may be substituted by $C_{1-3}$ alkyl, and $R_5$ is $C_{1-18}$ alkyl, $C_{3-8}$ cycloalkyl, phenylalkyl of phenyl which may be substituted by halogen, nitro and/or $C_{1-8}$ alkyl;

or its metal salt.

According to a second aspect of the present invention, there is provided an insecticidal composition comprising an inert carrier and an effective amount of the compound having the formula [I].

It is disclosed in U.S.P. 3,933,908, U.S.P. 4,005,223 or U.S.P. 4,085,226 the benzoylphenylurea derivatives having the formula

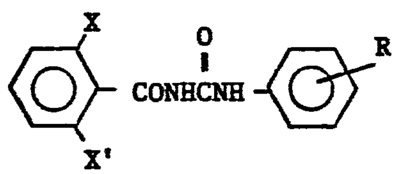

have an insecticidal activity.

As a commercial commodity of the said benzoylphenylurea derivative, "Diflubenzuron" disclosed in U.S.P. 3,933,908 is a typical merchandise.

The compound having the formula [I] has a superior insecticidal activity in comparison with the said known compounds and further, has a broader insecticidal spectrum and indicates a superior insecticidal activity or various kinds of injurious insects in Lepidoptera species, Coleoptera species, Diptera species or the like.

Furthermore, it has not only a larvicidal activity and an ovicidal activity but also it indicates the ovicidal effect to ovum under an oviposition in case of treating the insect adult with said compound.

The compounds has a low toxicity to haematothermal animals and it can be used in safety.

According to a third aspect of the invention, there is provided a process for the preparation of the compound of formula [I], comprising the step of reacting als illustrated by the following equations:

1. In case that A is Az group;

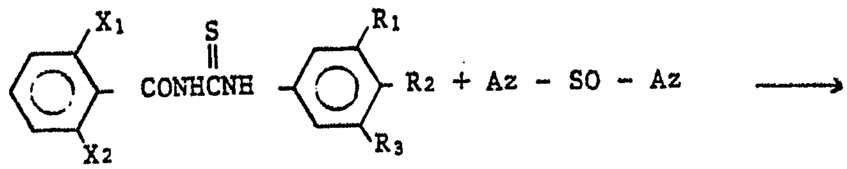

[II] [III]

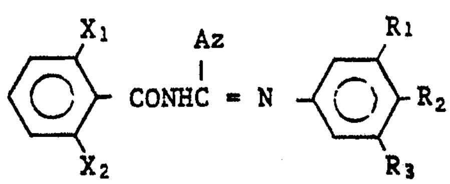

[I]'

The said compound having the formula [III] is produced by making a corresponding azole to react with thionyl chloride in an organic solvent.

Usually, the reaction compound is not separated from the organic solvent and is made to react with the said thiourea derivative having the formula [III].

As the organic solvent, a general inert solvent such as methylene dichloride, chloroform, acetonitrile or the like may be used.

The reaction temperature may be from 0°C tot 50°C, desirably 0°C to room temperature, and its reaction may be carried out for from 5 to 10 hours.

2. In case that A is $-SS-R_5$;

(a)

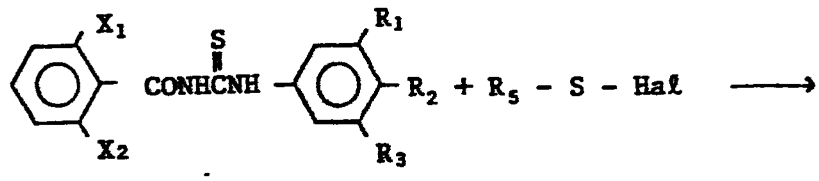

[II] [IV]

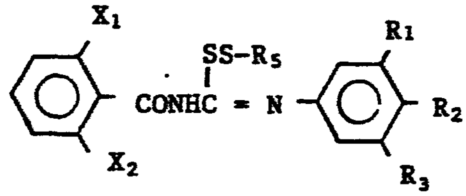

[I]"

wherein Hal is halogen.

The reaction may be conducted in an organic solvent in the presence of a base.

As the organic solvent, a general inert solvent such as benzene, toluene, dimethoxyethane, acetone, diethyl ether, THF or the like may be used and as the base, inorganic or organic base such as sodium alcoholate, sodium carbonate, triethylamine, dimethylaniline or the like may be used.

The reaction temperature may be from 0°C tot 50°C desirably, 0°C to room temperature, and its reaction may be carried out for from 5 to 10 hours.

(b)

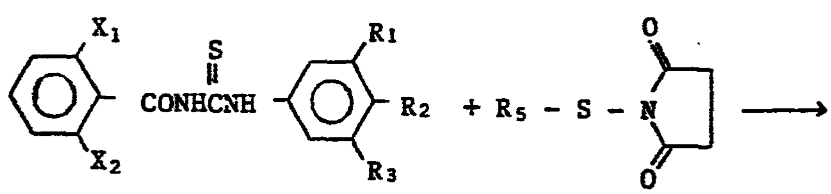

[II] [V]

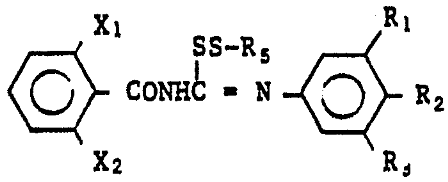

[I]"

The reaction may be conducted in an organic solvent in the presence of a catalyst.

As the organic solvent methylene dichloride, chloroform, acetonitrile or like may be used and as the catalyst, an organic base such as imidazole, triazole or the like may be used.

The reaction temperature may be from 0°C tot 50°C, desirably 0°C to room temperature, and its reaction may be carried out for 5 to 10 hours.

A chemical formula for the obtained compound can be assigned by means of IR spectrum, MASS spectrum and NMR spectrum.

In the event of the producing the sodium of potassium salts, a compound having the formula [I] may be mixed with a caustic alkali such as caustic soda and caustic potash in water or an inert organic solvent such as acetone, methanol, ethanol, dimethyl sulfoxide or the like, or mixed with metal sodium or metal potassium in alcohol solvent, and the resulting mixture is treated by heating if necessary, and thus its sodium salt or its potassium salt is obtained.

Other metal salt such as calcium, barium, manganese, copper, zinc, nickel, cobalt, iron and silver salt, may be prepared from the sodium or potassium salt by treatment with the appropiate inorganic metal salt, e.g. calcium chloride, barium chloride, copper sulfate, zink chloride, nickel chloride and cobalt nitrate.

In numerous cases, the metal salt of the compound having the formula [I] is precipitated or crystalized in above mentioned solvent.

It is expected that the compound having the formula [I] exsists in the following tautomeric forms shown as [I] and [I]" and further each tautomeric structure compound comprises "E isomer" and "Z isomer", and the present invention comprises all those isomers.

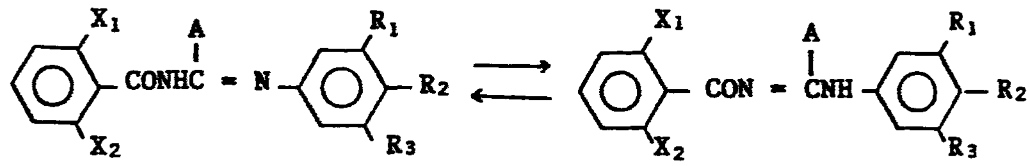

[I] [I]'''

The following Examples illustrate the invention.

Example 1

N-(2,6-difluorobenzoyl)-N'-(4-chloro-3-trifluoromethylphenyl)-1-imidazolecarboxyamidine (Compound No. 96):

1.5 g of thionylchloride was dropped into a solution of 3.5 g of imidazole in 20 ml of dried methylene dichloride under stirring and ice-water cooling.

4.5 g of N-(2,6-difluorobenzoyl)-N'-(4-chloro-3-trifluoromethylphenyl)thiourea was added to the mixture and the mixture was stirred at room temperature for 7 hours.

After the reaction, the insoluble material was filtered off and the filtrate was washed with water. The resulting precipitate was filtered and washed with diethyl ether to yield 3.4 g of the objective product. (m.p. 149-150°C)

Example 2

N-(2,6-difluorobenzoyl)-N'-(4-chloro-3-trifluoromethylphenyl)-1-benzimidazolecarboxyamidine (Compound No. 110):

Into 20 ml of dried methylene dichloride, 1.7 g of benzimidazole and 1.4 g of triethylamine, were added and dissolved. 0.83 g of thionyl chloride was dropped into the resulted solution under ice-water cooling and the mixture was stirred for 10 minutes at 0-5°C.

2.5 g of N-(2,6-difluorobenzoyl)-N'-(4-chloro-3-trifluoromethylphenyl)thiourea was added in it and the mixture was stirred at room temperature for 6 hours.

The insoluble material is filtered off and the filtrate was washed with water and dried with anhydrous magnesium sulfate.

The solvent of the dried solution was distilled off and then, the resulting crystal was washed with the mixed solvent of n-hexane

and diethyl ether (1:1 v/v) to yield 1.8 g of the objective product. (m.p. 168°-170°C)

Example 3

N-(2,6-difluorobenzoyl)-N'-(4-chloro-3-trifluoromethylphenyl)-1,2,4-triazole-1-yl-carboxyamidine (Compound No. 103):

Into 20 ml of dried methylene dichloride were dissolved 0.6 g of 1,2,4-triazole and 0.84 g of triethylamine, and 0.5 g of thionyl chloride was dropped to the solution under ice-water cooling and the mixture was stirred for 10 minutes at 0-5°C.

1.5 g of N-(2,6-difluorobenzoyl)-N'-(4-chloro-3-trifluoromethylphenyl)thiourea was added in the mixture and the resulted mixture was stirred at room temperature for 6 hours.

The insoluble material was filtered off and the filtrate was washed with water and dried with anhydrous magnesium sulfate.

The solvent of the dried solution was distilled off and the resulting crystal was washed with the mixed solvent of n-hexane and diethyl ether (1:1 v/v) to yield 0.9 g of the objective product. (m.p. 141°-143°C)

Example 4

N-(2,6-difluorobenzoyl)-N'-(4-chloro-3-trifluoromethylphenyl)-1-imidazolecarboxyamidine sodium salt (Compound No. 147):

0.1 g of sodium metal was dissolved in 20 ml of methanol and 2.0 g of N-(2,6-difluorobenzoyl)-N'-(4-chloro-3-trifluoromethylphenyl)-1-imidazolecarboxyamidine was added at once to the solution.

The solvent methanol was distilled off from the then obtained solution at reduced pressure, and the resulting crystal was washed with diethyl ether to yield 1.9 g of the objective product. (m.p. 174°-180°C)

Example 5

Bis-[N-(2,6-difluorobenzoyl)-N'-(4-chloro-3-trifluoromethylphenyl)-1-imidazolecarboxyamidine] manganese salt (Compound No. 149):

0.7 g N-(2,6-difluorobenzoyl)-N'-(4-chloro-3-trifluoromethylphenyl)-1-imidazolecarboxyamidine sodium salt was dissolved in 20 ml of water. To the solution was added a stoichiometrically excess amount of manganese acetate and the precipitate was gathered by filtration and washed with water, and then dried in a desiccator to yield 0.5 g of the objective product. (m.p. 180°-184°C)

Example 6

Bis-[N-(2,6-difluorobenzoyl)-N'-(3-chloro-4-trifluoromethylphenyl)-1-imidazolecarboxyamidine] calcium salt (Compound No. 164):

1.5 g N-(2,6-difluorobenzoyl)-N'-(3-chloro-4-trifluoromethylphenyl)-1-imidazolecarboxyamidine sodium salt was dissolved in 30 ml of water. To the solution was added a stoichiometrically excess amount of calcium chloride and the precipitate was filtered, washed with water and dried in a desiccator to yield 1.3 g of the title product. (m.p. 173°-176°C)

Example 7

N-(2,6-difluorobenzoyl)-N'-(4-chloro-3-trifluoromethylphenyl)-(4-methylphenyl)dithiocarboxyamidine (Compound No. 214):

To a suspension of 1.0 g of N-(2,6-difluorobenzoyl)-N'-(4-chloro-3-trifluoromethylphenyl)thiourea in 20 ml of a dried dichloromethane were added 0.56 g of N-4-methyl-phenylsulfenylsuccinimide and a catalytic amount of imidazole, and the mixture was stirred at room temperature for 5 hours.

The reaction mixture was washed with water and dried with anhydrous magnesium sulfate.

The solvent was distilled off at a reduced pressure from the dried solution after filtration, and the remaining crude crystal was washed with n-hexane to yield 1.0 g of the desired product. (m.p. 99°-101°C)

Example 8

N-(2,6-difluorobenzoyl)-N'-(4-chloro-3-trifluoromethylphenyl)-ethyldithiocarboxyamidine (Compound No. 205):

To a suspension of 1 g of N-(2,6-difluorobenzoyl)-N'-(4-chloro-3-trifluoromethylphenyl)thiourea in 20 ml of dichloromethane were added 0.46 g of N-ethylsulfenylsuccinimide and a catalytic amount of imidazole, and the mixture was stirred at room temperature for 5 hours.

The reaction mixture was washed with water and dried with anhydrous magnesium sulfate.

The solvent was distilled off at a reduced pressure from the dried solution after filtration, and the remaining crude product was washed with n-hexane to yield 0.9 g of the desired product. (m.p. 83°-85°C)

Example 9

N-(2,6-difluorobenzoyl)-N'-(4-chloro-3-trifluoromethylphenyl)-(4-nitrophenyl) dithiocarboxyamidine (Compound No. 220):

To a suspension of 1 g of N-(2,6-difluorobenzoyl)-N'-(4-chloro-3-trifluoromethylphenyl)thiourea in 30 ml dichloromethane were added 0.25 g of triethylamine and 0.48 g of N-nitrophenylsulfenylchloride under ice-water cooling, and the mixture was stirred at room temperature for 30 minutes.

The reaction mixture was washed with water and dried with anhydrous magnesium sulfate.

The solvent was distilled off at a reduced pressure from the dried solution after filtration, and the remaining crude crystal was washed with a mixed solvent of n-hexane and diethyl ether to yield 0.8 g of the desired product. (m.p. 169°-175°C)

Inclusive of the above, each compound within the scope of this invention which can be prepared in an analogeous manner is tabulated in Table 1.

Table 1

1-1

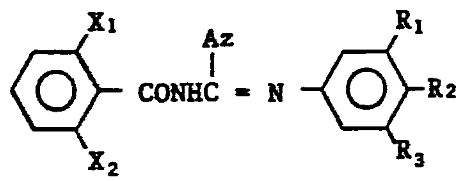

| Compound No. | $X_1$ | $X_2$ | Az | $R_1$ | $R_2$ | $R_3$ | Physical Properties [m.p.]°C |
|---|---|---|---|---|---|---|---|
| 1 | F | F | 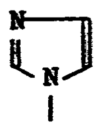 | H | $-NO_2$ | H | [145-146] |
| 2 | " | " | " | " | $-CH_3$ | " | [160-161] |
| 3 | " | " | " | " | $-C_2H_5$ | " | [139-142] |
| 4 | " | " | " | " | $-C_3H_7^n$ | " | [148,5-151,5] |
| 5 | " | " | " | " | $-C_4H_9^n$ | " | [139-142] |
| 6 | " | " | " | " | $-C_4H_9^t$ | " | [147-150] |
| 7 | " | " | " | " | -N=N- 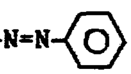 | " | [173-175] |
| 8 | " | " | " | Cl | $-CH_3$ | " | [152-154] |
| 9 | " | " | " | " | $-OCH_3$ | " | [157-158] |
| 10 | " | " | " | " | H | Cl | [144-146] |
| 11 | Cl | H | " | " | Cl | " | [145-147] |
| 12 | Br | " | " | " | " | " | [140-143] |
| 13 | $CH_3$ | " | " | " | " | " | [135-138] |
| 14 | F | F | " | " | " | " | [139-142] |
| 15 | " | " | 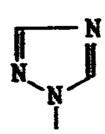 | " | " | " | [157-160] |

1-1

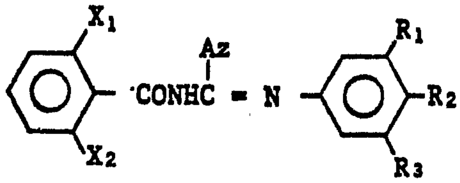

| Compound No. | $X_1$ | $X_2$ | Az | $R_1$ | $R_2$ | $R_3$ | Physical Properties [m.p.]°C |
|---|---|---|---|---|---|---|---|
| 16 | F | F | 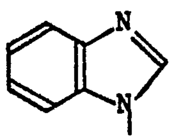 | Cl | Cl | Cl | [166-169] |
| 17 | " | " | 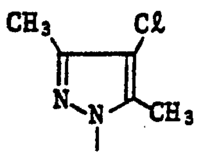 | " | " | " | [156-159] |
| 18 | " | " | 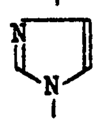 | " | F | " | [152-153,5] |
| 19 | " | " | " | " | $-CH_3$ | " | [154-156] |
| 20 | " | " | " | " | $-C_3H_7{}^n$ | " | [141-143] |
| 21 | " | " | " | " | Cl | $CF_3$ | [118-121] |
| 22 | Cl | H | " | " | " | " | [134-135] |
| 23 | F | F | " | " | $-SC_2H_5$ | Cl | [127-129] |
| 24 | " | " | " | " | 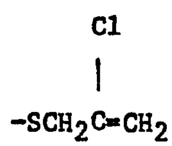 | " | [126-127] |
| 25 | " | " | " | " | 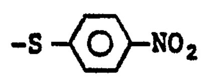 | " | [116-118] |
| 26 | " | " | " | " | $N(C_2H_5)_2$ | " | [127-131] |
| 27 | " | " | " | " | $-OC_2H_5$ | " | [128-130] |
| 28 | " | " | " | " | $-OC_6H_{13}{}^n$ | " | [123-124] |

1-1

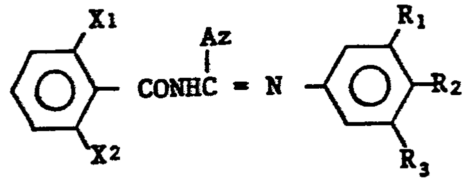

| Compound No. | $X_1$ | $X_2$ | Az | $R_1$ | $R_2$ | $R_3$ | Physical Properties [m.p.]°C |
|---|---|---|---|---|---|---|---|
| 29 | F | F | 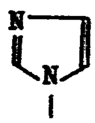 | Cl | $-OCH_2CH_2F$ | Cl | [120-122] |
| 30 | " | " | " | " | $-OCHF_2$ | " | [148-150] |
| 31 | " | " | " | " | $-OCF_2CF_2H$ | " | [122-125] |
| 32 | " | " | " | " | $-OCF_2CClFH$ | " | [137-140] |
| 33 | " | " | " | Br | $-OCF_2CF_2H$ | " | [130-132,5] |
| 34 | " | " | 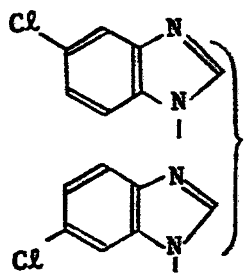 (mixture) | Cl | " | " | [182-186] |
| 35 | " | " | 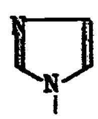 | " | $-OCH_2CH{=}CH_2$ | " | [114-115] |
| 36 | Cl | H | " | " | " | " | [121-123] |
| 37 | F | F | 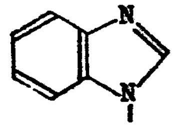 | " | $-OCH_2CH_2CH{=}CH_2$ | " | [120-124] |
| 38 | " | " | 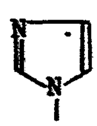 | " | " | " | [131-133] |
| 39 | " | " | " | " | $-OCH_2CH{=}CHCH_3$ | " | [146-148] |

| Compound No. | 1-1 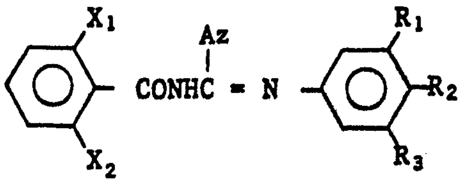 | | | | | | Physical Properties [m.p.]°C |
|---|---|---|---|---|---|---|---|
| | $X_1$ | $X_2$ | Az | $R_1$ | $R_2$ | $R_3$ | |
| 40 | F | F | 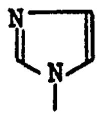 | Cl | $-OCH_2CH=CHCl$ | Cl | [125-127] |
| 41 | " | " | 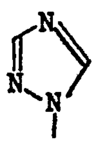 | " | " | " | [147-148] |
| 42 | " | " | 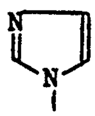 | " | Cl<br>\|<br>$-OCH_2C=CH_2$ | " | [131-133] |
| 43 | " | " | " | " | $-OCH_2CH=CCl_2$ | " | [138-139] |
| 44 | " | " | " | " | 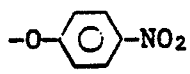 | " | [110-113] |
| 45 | " | " | " | " | 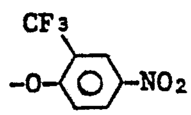 | " | [130-135] |
| 46 | " | " | " | " | 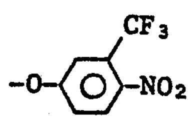 | " | [164-166] |
| 47 | " | " | " | " | 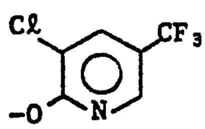 | " | [115-119] |
| 48 | " | " | " | " | 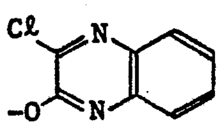 | " | [168-172] |

1-1

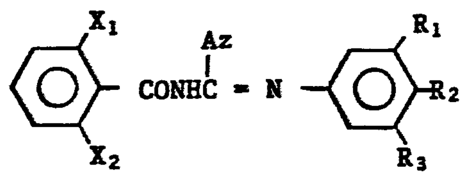

| Compound No. | $X_1$ | $X_2$ | Az | $R_1$ | $R_2$ | $R_3$ | Physical Properties [m.p.]°C |
|---|---|---|---|---|---|---|---|
| 49 | F | F | 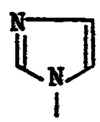 | Cl | 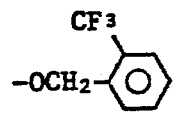 | Cl | [137-139] |
| 50 | " | " | " | " | 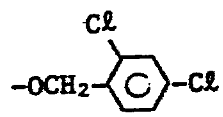 | " | [139-141] |
| 51 | " | " | " | " | 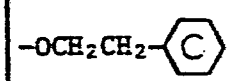 | " | [135-137] |
| 52 | " | " | " | " | 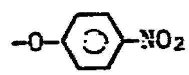 | H | [136-140] |
| 53 | " | " | " | $CF_3$ | H | $CF_3$ | [127-130] |
| 54 | " | " | " | " | 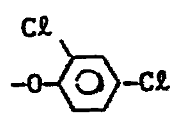 | H | [124-126] |
| 55 | " | " | " | H | Cl | " | [160-161] |
| 56 | Cl | Cl | " | " | " | " | [202-205] |
| 57 | " | " | 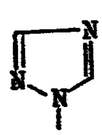 | " | " | " | [197-199] |

1-1

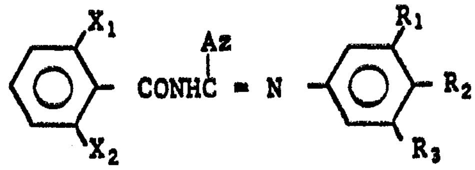

| Compound No. | $X_1$ | $X_2$ | Az | $R_1$ | $R_2$ | $R_3$ | Physical Properties [m.p.]°C |
|---|---|---|---|---|---|---|---|
| 58 | Cl | Cl | 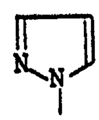 | H | Cl | H | [138-144] |
| 59 | " | " | 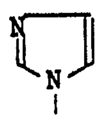 | " | F | " | [180-185] |
| 60 | " | H | " | " | " | " | [154-155] |
| 61 | F | F | " | " | $CF_3$ | " | [141-143] |
| 62 | Cl | Cl | " | " | " | " | [204-205] |
| 63 | F | F | " | " | $OCF_3$ | " | [133-134] |
| 64 | Cl | H | " | " | " | " | [139-141] |
| 65 | Br | " | " | " | $-OCF_3$ | " | [143-145] |
| 66 | F | F | " | " | $-OCF_2Br$ | " | [142-143] |
| 67 | " | " | " | " | $-OCF_2CF_2H$ | " | [148-150] |
| 68 | " | " | " | " | $-OCF_2CFClH$ | " | [151-153,5] |
| 69 | $CH_3$ | H | " | " | $-OCF_3$ | " | [112.5-115] |
| 70 | F | F | " | Cl | Cl | " | [150-152] |
| 71 | Cl | H | " | " | " | " | [145-147] |
| 72 | " | Cl | " | " | " | " | [177-183] |
| 73 | " | " | 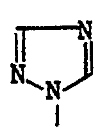 | " | " | " | [179-182] |
| 74 | " | H | " | " | " | " | [156-157] |

1-1

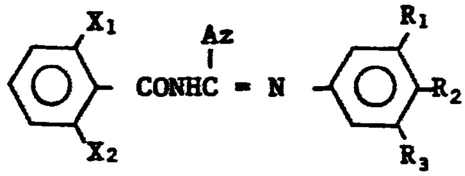

| Compound No. | $X_1$ | $X_2$ | Az | $R_1$ | • $R_2$ | $R_3$ | Physical Properties [m.p.]°C |
|---|---|---|---|---|---|---|---|
| 75 | F | F | 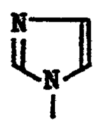 | $NO_2$ | Cl | H | [128-131] |
| 76 | Cl | Cl | 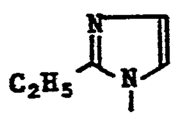 | Cl | " | " | [129-133] |
| 77 | F | F | 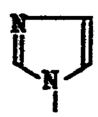 | " | F | " | [165] |
| 78 | " | " | " | " | $CF_3$ | " | [106-109] |
| 79 | Cl | H | " | " | " | " | [143-145] |
| 80 | Cl | Cl | " | " | " | " | [185-187] |
| 81 | F | F | 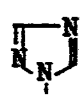 | " | " | " | [109-112] |
| 82 | " | " | 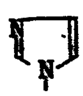 | " | $OCF_3$ | " | [137-139] |
| 83 | Cl | Cl | " | " | $OCF_3$ | " | [162-164] |
| 84 | " | F | " | " | " | " | [167-169] |
| 85 | " | H | " | " | " | " | [115.5-118] |
| 86 | Br | " | " | " | " | " | [116-118] |
| 87 | F | " | " | " | " | " | [92-97] |
| 88 | $CH_3$ | " | " | " | " | " | [129-131] |

1-1

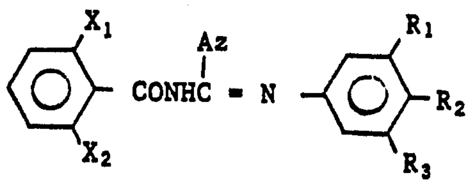

| Compound No. | $X_1$ | $X_2$ | Az | $R_1$ | $R_2$ | $R_3$ | Physical Properties [m.p.]°C |
|---|---|---|---|---|---|---|---|
| 89 | F | F | 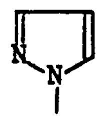 | $NO_2$ | $OCF_3$ | H | [102-105] |
| 90 | " | " | 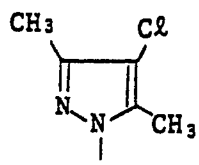 | Cl | " | " | [133-136] |
| 91 | " | " | 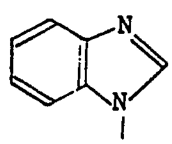 | " | " | " | [178-181] |
| 92 | " | " | 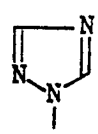 | " | " | " | [108-111] |
| 93 | " | " | 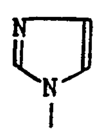 | " | $-OCF_2CF_2H$ | " | [113-116] |
| 94 | Cl | H | " | $CF_3$ | H | " | [126-127] |
| 95 | F | F | " | " | " | " | [139-141] |
| 96 | " | " | " | " | Cl | " | [149-150] |
| 97 | Cl | Cl | " | " | " | " | [177-180] |
| 98 | F | H | " | " | " | " | [113-115] |
| 99 | Cl | " | " | " | " | " | [128-129] |
| 100 | Br | " | " | " | " | " | [126-128] |
| 101 | $CH_3$ | " | " | " | " | " | [127-129] |
| 102 | F | Cl | " | " | " | " | [156-158] |

1-1

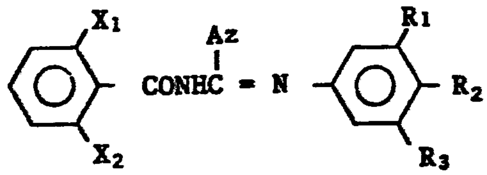

| Compound No. | $X_1$ | $X_2$ | Az | $R_1$ | $R_2$ | $R_3$ | Physical Properties [m.p.]°C |
|---|---|---|---|---|---|---|---|
| 103 | F | F | 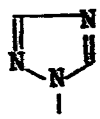 | $CF_3$ | Cl | H | [141-143] |
| 104 | " | Cl | " | " | " | " | [103-107] |
| 105 | " | F | 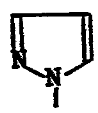 | " | " | " | [109-111] |
| 106 | " | " | 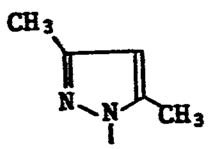 | " | " | " | [165-167] |
| 107 | " | " | 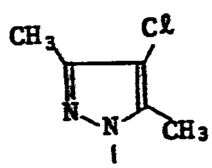 | " | " | " | [137-139] |
| 108 | " | " | 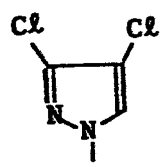 | " | " | " | [121-123] |
| 109 | " | " | 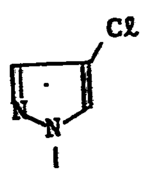 | " | " | " | [119-122] |

| Compound No. | 1-1 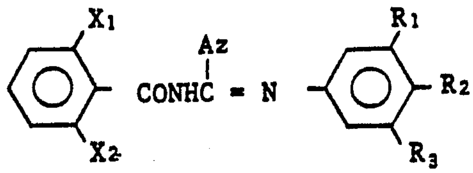 | | | | | | Physical Properties [m.p.]°C |
|---|---|---|---|---|---|---|---|
| | $X_1$ | $X_2$ | Az | $R_1$ | $R_2$ | $R_3$ | |
| 110 | F | F | 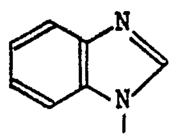 | $CF_3$ | Cl | H | [168-170] |
| 111 | " | " | 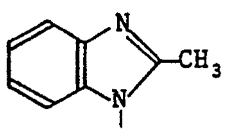 | " | " | " | [143-144] |
| 112 | " | " | 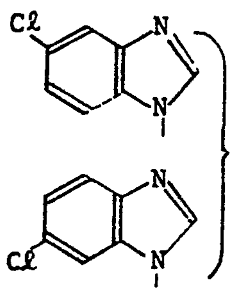 (mixture) | " | " | " | [143-147] |
| 113 | " | " | 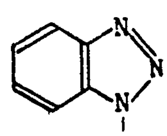 | " | " | " | [154-157] |
| 114 | " | " | 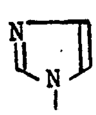 | " | Br | " | [146-147.5] |
| 115 | " | Cl | " | " | " | " | [162-163] |
| 116 | " | F | " | " | F | " | [137-139] |

1-1

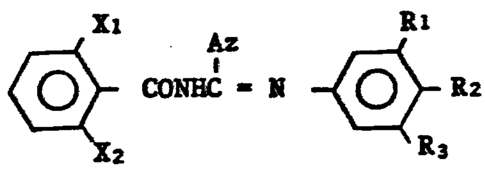

| Compound No. | $X_1$ | $X_2$ | Az | $R_1$ | $R_2$ | $R_3$ | Physical Properties [m.p.]°C |
|---|---|---|---|---|---|---|---|
| 117 | F | F | 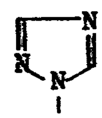 | $CF_3$ | Br | H | [144-147] |
| 118 | " | " | 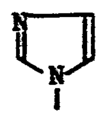 | " | $-OCH_2CF_3$ | " | [110-113] |
| 119 | " | " | " | Cl | $-OCF_3$ | Cl | [137-139] |
| 120 | " | " | " | " | $-OCF_2CCl_2H$ | " | [144-146] |
| 121 | " | " | " | " | F | $CF_3$ | [127-130] |
| 122 | $CH_3$ | H | " | $CF_3$ | Br | H | [124-125] |
| 123 | " | " | 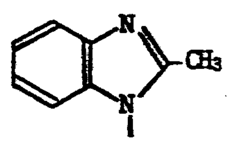 | " | " | " | [135-138] |
| 124 | " | " | 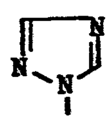 | " | " | " | [153-156] |
| 125 | " | " | 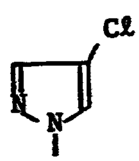 | " | " | " | [126-128] |
| 126 | " | " | 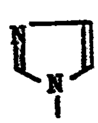 | H | $CF_3$ | " | [129-130] |
| 127 | Cl | H | " | $CF_3$ | F | Cl | [137-140] |

1-1

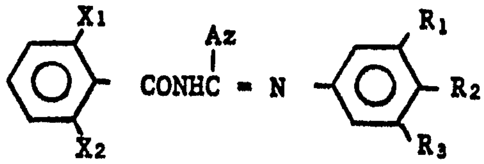

| Compound No. | $X_1$ | $X_2$ | Az | $R_1$ | $R_2$ | $R_3$ | Physical Properties [m.p.]°C |
|---|---|---|---|---|---|---|---|
| 128 | F | F | " | $CF_3$ | Cl | Br | [129-132] |
| 129 | Cl | H | " | " | " | " | [146-149] |
| 130 | F | F | 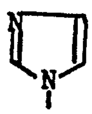 | " | F | Br | [133-137] |
| 131 | " | " | " | H | 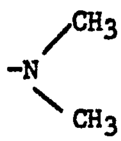 | H | [159-163] |
| 132 | " | " | " | " | 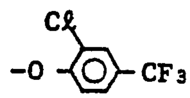 | H | [130-132] |
| 133 | " | " | " | Cl | $OCH_2CF_3$ | $CF_3$ | [135-137] |
| 134 | Cl | H | " | " | " | " | [128-129] |
| 135 | F | F | " | " | 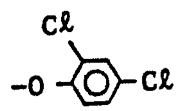 | " | [141-144] |
| 136 | " | " | " | H | $-COOC_4H_9{}^t$ | H | [180-183] |
| 137 | $CH_3$ | H | " | $CF_3$ | Cl | Br | [119-122] |
| 138 | " | " | 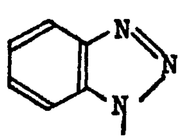 | " | Br | H | [130-133] |
| 139 | Cl | " | 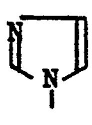 | Cl | 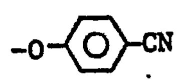 | " | [130-135] |
| 140 | F | F | " | Cl | $-OCH_2C{\equiv}CH$ | Cl | [109-111] |

| Compound No. | 1-1 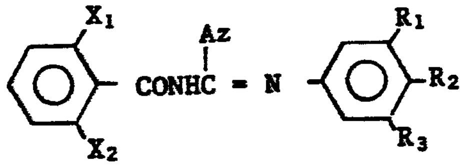 | | | | | | Physical Properties [m.p.]°C |
|---|---|---|---|---|---|---|---|
| | $X_1$ | $X_2$ | Az | $R_1$ | $R_2$ | $R_3$ | |
| 141 | F | F | 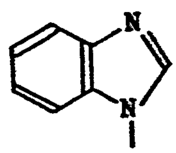 | Cl | $CF_3$ | H | [142-146] |
| 142 | " | " | 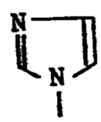 | $CF_3$ | 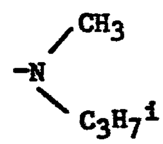 | " | [123-126] |
| 143 | " | " | " | H | H | 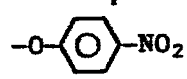 | [153-154] |
| 144 | " | " | " | " | " | 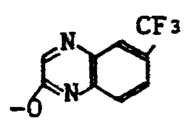 | [118-111] |
| 145 | " | " | " | Cl | 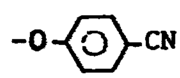 | H | [136-138] |
| 146 | " | " | " | H | H | $OCH_2CH=CH_2$ | [70-75] |

1-2

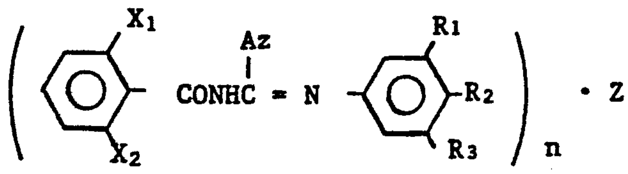

| Compound No. | $X_1$ | $X_2$ | Az | $R_1$ | $R_2$ | $R_3$ | n | Z | Physical Properties [m.p.]°C |
|---|---|---|---|---|---|---|---|---|---|
| 147 | F | F | 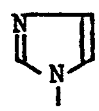 | $CF_3$ | Cl | H | 1 | Na | [174-180] |
| 148 | " | " | " | " | " | " | 2 | Ca | [163-165] |
| 149 | " | " | " | " | " | " | " | Mn | [180-184] |
| 150 | " | " | " | " | " | " | " | Zn | [176-181] |
| 151 | " | " | " | " | " | " | 1 | K | [172-177] |
| 152 | " | " | " | " | " | " | 2 | Cu | [241-244] |
| 153 | " | " | " | " | " | " | " | Mg | [179-184] |
| 154 | " | " | " | " | " | " | 3 | Al | [153-154] |
| 155 | " | " | " | " | " | " | 2 | Fe | [144-147] |
| 156 | " | " | " | " | " | " | " | Sn | [143-144] |
| 157 | " | " | 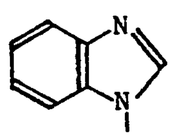 | " | " | " | 1 | Na | [158-162] |
| 158 | " | " | 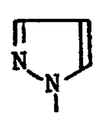 | " | " | " | " | " | [180-184] |
| 159 | " | " | 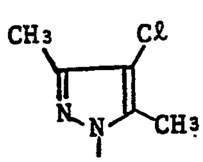 | " | " | " | " | " | [157-160] |

1-2

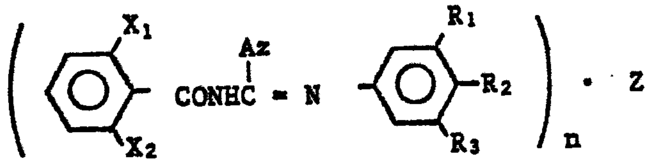

| Compound No. | $X_1$ | $X_2$ | Az | $R_1$ | $R_2$ | $R_3$ | n | Z | Physical Properties [m.p.]°C |
|---|---|---|---|---|---|---|---|---|---|
| 160 | F | F | 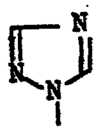 | $CF_3$ | Cl | H | 1 | Na | [165-170] |
| 161 | " | " | 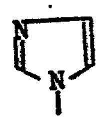 | Cl | $OCF_3$ | " | " | K | [130-133] |
| 162 | H | Cl | " | " | " | " | " | Na | [160-164] |
| 163 | F | F | " | " | " | " | " | " | [178-182] |
| 164 | " | " | " | " | " | " | 2 | Ca | [173-176] |
| 165 | " | " | " | " | " | " | " | Mn | [177-180] |
| 166 | H | Cl | " | H | " | " | 1 | Na | [183-185] |
| 167 | " | " | " | " | " | " | 2 | Mn | [158-160] |
| 168 | F | F | 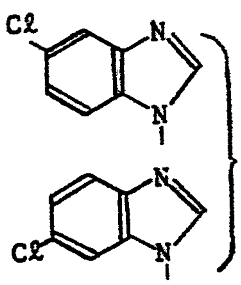 (mixture) | $CF_3$ | Cl | " | 1 | Na | [161-164] |
| 169 | " | " | 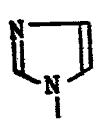 | Cl | 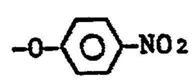 | " | 1 | Na | [170-175] |

1-2

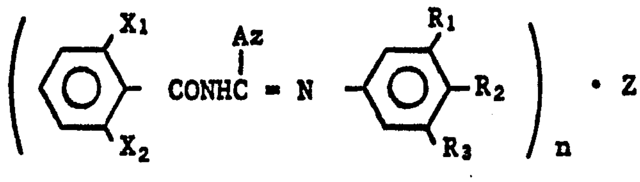

| Compound No. | $X_1$ | $X_2$ | Az | $R_1$ | $R_2$ | $R_3$ | n | Z | Physical Properties [m.p.]°C |
|---|---|---|---|---|---|---|---|---|---|
| 170 | F | F | 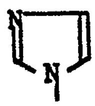 | Cl | Cl | Cl | 1 | Na | [173-176] |
| 171 | Br | H | " | " | " | " | " | " | [179-183] |
| 172 | F | F | " | " | " | " | 2 | Ca | [166-169] |
| 173 | " | " | " | " | " | " | " | Cu | [173-175] |
| 174 | " | " | " | " | " | " | " | Mn | [143-146] |
| 175 | " | " | " | " | " | " | " | Zn | [162-165] |
| 176 | " | " | 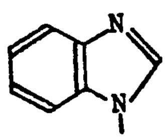 | " | " | " | 1 | Na | [177-180] |
| 177 | " | " | " | " | " | " | 2 | Mn | [186-189] |
| 178 | " | " | 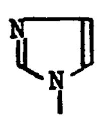 | " | $OCF_2CF_2H$ | " | 1 | Na | [157-161] |
| 179 | " | " | " | " | $OCH_2CH_2CH{=}CH_2$ | " | " | " | [140-143] |
| 180 | " | " | 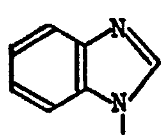 | " | " | " | " | " | [148-152] |
| 181 | " | " | 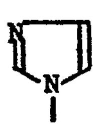 | " | OCH2CH=CHCl | " | " | " | [139-141] |

1-2

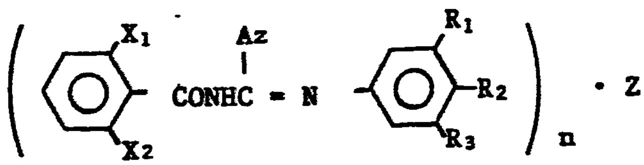

| Compound No. | $X_1$ | $X_2$ | Az | $R_1$ | $R_2$ | $R_3$ | n | Z | Physical Properties [m.p.]°C |
|---|---|---|---|---|---|---|---|---|---|
| 182 | $CH_3$ | H | 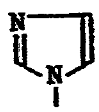 | $CF_3$ | Br | H | 1 | Na | [170-175] |
| 183 | " | " | " | " | " | " | 3 | Al | [78-81] |
| 184 | " | " | " | H | $-CF_3$ | " | 1 | Na | [184-187] |
| 185 | " | " | " | " | " | " | 2 | Sn | |
| 186 | Cl | " | " | Cl | $-OCF_3$ | " | " | Mn | [164-168] |
| 187 | Br | " | " | " | Cl | Cl | " | " | [174-178] |
| 188 | " | " | " | " | " | " | " | Zn | [153-157] |
| 189 | F | F | 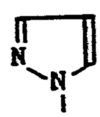 | $CF_3$ | " | H | " | Mn | [206-211] |
| 190 | " | " | 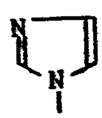 | Cl | $-OCH_2CH_2CH=CH_2$ | Cl | " | " | [148-152] |
| 191 | " | " | " | " | $CF_3$ | H | 1 | Na | [176-180] |

1-3

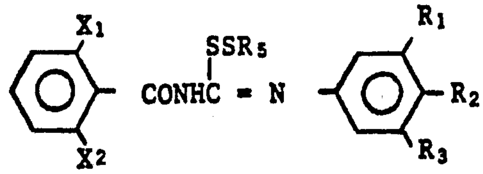

| Compound No. | $X_1$ | $X_2$ | $R_5$ | $R_1$ | $R_2$ | $R_3$ | Physical Properties [m.p.]°C |
|---|---|---|---|---|---|---|---|
| 192 | F | F | $-C_2H_5$ | H | $-CF_3$ | H | $n_D^{25}$ 1.5913 |
| 193 | " | " | 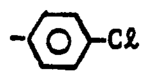 | " | " | " | [108-111] |
| 194 | " | " | $-C_2H_5$ | " | $-OCF_3$ | " | $n_D^{28}$ 1.5818 |
| 195 | Cl | H | 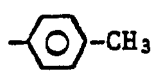 | " | " | " | [92-94] |
| 196 | F | F | " | " | " | " | $n_D^{27.5}$ 1.6104 |
| 197 | " | " | " | " | $NO_2$ | " | [103-107] |
| 198 | " | " | 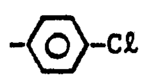 | Cl | Cl | " | [112-113] |
| 199 | " | " | 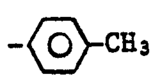 | " | " | Cl | [123-124] |
| 200 | " | " | " | " | $CF_3$ | H | [71-74] |
| 201 | " | " | $-C_3H_7^n$ | " | $OCF_3$ | " | [86-87] |
| 202 | Br | H | " | " | " | " | [69-71] |

1-3

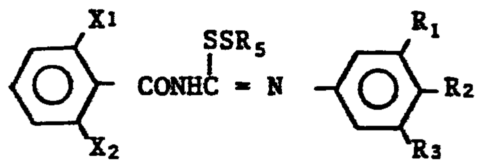

| Compound No. | $X_1$ | $X_2$ | $R_5$ | $R_1$ | $R_2$ | $R_3$ | Physical Properties [m.p.]°C |
|---|---|---|---|---|---|---|---|
| 203 | F | F | 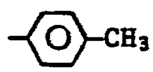 | Cl | $OCF_3$ | H | [77–80] |
| 204 | " | " | " | $CF_3$ | H | " | $n_D^{25.5}$ 1.6195 |
| 205 | " | " | $-C_2H_5$ | " | Cl | " | [83–85] |
| 206 | $CF_3$ | H | " | " | " | " | [71–73] |
| 207 | F | F | $C_3H_7^n$ | " | " | " | [88–90] |
| 208 | " | " | $-C_3H_7^i$ | " | " | " | [101–105] |
| 209 | " | " | $-C_6H_{13}^n$ | " | " | " | [87–88] |
| 210 | " | " | $-C_{12}H_{25}^n$ | " | " | " | $n_D^{27}$ 1.5560 |
| 211 | " | " | 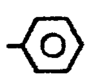 | " | " | " | [92–93] |
| 212 | " | " | 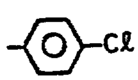 | " | " | " | [90–94] |
| 213 | " | " | 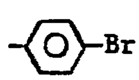 | " | " | " | [99–103] |
| 214 | " | " | 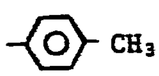 | " | " | " | [99–101] |

1-3

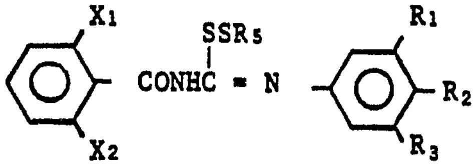

| Compound No. | $X_1$ | $X_2$ | $R_5$ | $R_1$ | $R_2$ | $R_3$ | Physical Properties [m.p.]°C |
|---|---|---|---|---|---|---|---|
| 215 | F | F | 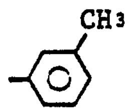 | $CF_3$ | Cl | H | [97-99] |
| 216 | " | " | 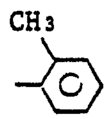 | " | " | " | [83-86] |
| 217 | " | Cl | 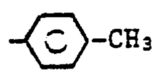 | " | " | " | [82-85] |
| 218 | $CF_3$ | H | " | " | " | " | [104-107] |
| 219 | F | F | 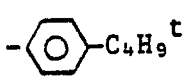 | " | " | " | [84-87] |
| 220 | " | " | 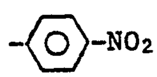 | " | " | " | [169-175] |
| 221 | " | " | 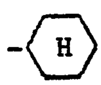 | " | " | " | [94-98] |
| 222 | " | " | 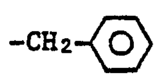 | " | " | " | [100-102] |

1-3

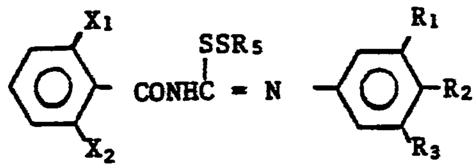

| Compound No. | $X_1$ | $X_2$ | $R_5$ | $R_1$ | $R_2$ | $R_3$ | Physical Properties [m.p.]°C |
|---|---|---|---|---|---|---|---|
| 223 | F | F | 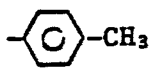 | $CF_3$ | Br | H | [82-85] |
| 224 | " | " | $C_3H_7$i | Cl | $-OCF_2CF_2H$ | Cl | [107-108] |
| 225 | " | " | " | " | $-OCH_2\overset{Cl}{\overset{\vert}{C}}=CH_2$ | " | [105-108] |
| 226 | " | " | " | " | $-SC_2H_5$ | " | [136-139.5] |
| 227 | " | " | " | " | $-SCH_2\overset{Cl}{\overset{\vert}{C}}=CH_2$ | " | [85-87] |
| 228 | " | " | " | " | $CF_3$ | H | [108-111] |

As already mentioned, the compound having the formula [I] exhibits an outstanding insecticidal efficacy and an insecticidal composition containing the compound as an active ingredient may be formulated by mixing suitable carrieres in a form generally used in agricultural pesticide, such as wettable powder, emulsifiable concentrate, water-soluble powder, dust, granular formulation, suspension concentrate or the like.

As solid carriers, cereal flours such as soy bean flour and wheat flour, ground minerals such as diatomaceous earth, apatite, gypsum, talc, bentonite and clay, and organic or inorganic compounds such as sodium benzoate, urea and sodium sulfate may be used.

As liquid carriers, vegetable oli, mineral oil, petroleum such as kerosine solvent naphta and xylene, cyclohexane, cyclohexanone, dimethylformamide, dimethylsulfoxide, trichloroethylene, methylisobutyl ketone and water may be used.

A surfactant may, if necessary, be added in order to give a homogeneous and stable formulation.

The concentration of the active ingredient in an insecticidal composition may vary according to type of formulation, and is, for example, in the range of 5-70 weight percent, preferably 10-30 weight percent, in wettable powder; 5-30 weight percent, preferably 10-20 weight percent, in emulsifiable concentrate; 5-80 weight percent, preferably 30-60 weight percent, in water soluble-powder; 1-10 weight percent, preferably 2-5 weight percent in dust; 5-40 weight percent, preferably 10-30 weight percent in suspension concentrate; 1-10 weight percent, preferably 2-5 weight percent in granular formulation.

The wettable powder, the emulsifiable concentrate, water soluble-powder and suspension concentrate are diluted with water to the specified concentrations and used as a liquid suspension or a liquid emulsion to spray over the plants.

The dust and granular formulation are directly used for spraying over the plants.

Examples of the insecticidal composition of this invention are as mentioned below, but the scope of the invention shall not be limited to those:

Example 10 Emulsifiable Concentrate:

| | | |
|---|---|---|
| Compound of this invention | 10 | parts by weight |
| Calcium dodecylbenzenesulfonate | 5 | " |
| Dimethylformamide | 40 | " |
| Xylene | 40 | " |
| Polyoxyethylene styrylphenyl ether | 3 | " |
| Polyoxyethylene alkylaryl ether | 2 | " |

Those are mixed together to provide an emulsifiable concentrate containing 10% of active ingredient. At use, it is diluted with water to the desired concentration of the emulsion.

Example 11 Wettable powder:

| | | |
|---|---|---|
| Compound of this invention | 20 | parts by weight |
| Talc | 75 | " |
| Sodium lignin sulfonate | 3 | " |
| Sodium stearate | 2 | " |

Those are mixed together to provide a wettable powder containing 20% of active ingredient. At use, it is diluted with water to the desired concentration of the suspension.

Example 12 Water Soluble Powder:

| | | |
|---|---|---|
| Compound of this invention | 50 | parts by weight |
| Sodium alkylsulfosuccinate | 10 | " |
| Sodium benzoate | 40 | " |

Those are mixed to provide a water-soluble powder containing 50% of active ingredient. At use, it is diluted with water to obtain a solution or a suspension at the desired concentration.

Example 13 Dust:

| | | |
|---|---|---|
| Compound of this invention | 5 | parts by weight |
| Talc | 92 | " |
| Silicone | 3 | " |

Those are mixed together to provide a dust containing 5% of active ingredient.

Further, it goes without saying that the compound indicates a sufficient insecticidal efficacy, but, in the insecticidal composition, one kind or two kinds or more of other insecticidal compounds may be mixed in order to give a rapid insecticidal action or extend its spectrum (hereinafter called as "mixed composition"), because the compound of this invention indicates a slow-acting effect on larva or lacks the sufficient insecticidal effect against adult insect.

In the mixed composition, the compound can be used with one kind or two kinds or more of fungicidal and/or acaricidal compound(s) as well as the insecticidal compound having the rapid action.

Typical examples of insecticidal compounds usable together with the compound of this invention in the mixed composition as active ingredient(s) are set forth below:

(Organophosphorous compounds or carbamates)

fenthion, fenitrotion, diazinon, chlorpyrifos, ESP, vamidothion, phenthoate, dimethoate, formotion, malathion, trichlorfon, thiomethon, dichlorvos, acephate, cyanophos, pyrimiphos methyl, isoxathin, pyridaphenthion, DMTP, prothiophos, sulprofos, profenofos, CVMP, salithion, EPN, CYP, aldicarb, propoxur, pyrimicarb, methomyl, cartap, carbaryl, thiodicarb, carbosulfen, carbosulfan, nicotine.

(Pyrethroids)

permethrin, cypermethrin, decamethrin, fenvalerate, fenpropathrin, cyhalothrin, flucythrate, fencyclate, tetramethrin, cyfluthrin, fluvalinate, pyrethrin, allethrin, tetramethrin, resmethrin, barthrin, dimethrin, propathrin, prothrin.

The insecticidal activity of the compound is illustrated by the following tests.

Test 1 Insecticidal activity against Armyworm:

An emulsifiable concentrate or a wettable powder (in case of metal salt of the compound) formulated according to the aforesaid Examples was diluted with water to the concentrations of 500 ppm and 125 ppm of the compound. A leaf of corn was immersed in the liquid formulation for 30 seconds and air-dries. The treated leaf was put in a petri dish enclosing 5 third instar larvae of the armyworm, and the petri dish was capped with a sheet of glass. The petri dish was placed in a room kept at 25°C and 65% relative humidity, and the mortality was investigated after 120 hours. The results obtained in two replicates are shown in Table 2.

Table 2

| Compound No. | Mortality after 120 hours | |
|---|---|---|
| | 500 ppm | 125 ppm |
| 1 | 100% | 100% |
| 4 | 100 | 100 |
| 5 | 100 | 100 |
| 6 | 100 | 100 |
| 7 | 100 | 100 |
| 10 | 100 | 100 |
| 11 | 100 | 100 |
| 14 | 100 | 100 |
| 15 | 100 | 100 |
| 16 | 100 | 100 |
| 17 | 100 | 100 |
| 18 | 100 | 100 |
| 20 | 100 | 100 |
| 21 | 100 | 100 |
| 22 | 100 | 100 |
| 25 | 100 | 100 |
| 30 | 100 | 100 |
| 31 | 100 | 100 |
| 32 | 100 | 100 |
| 33 | 100 | 100 |
| 34 | 100 | 100 |
| 35 | 100 | 100 |
| 37 | 100 | 100 |
| 38 | 100 | 100 |
| 39 | 100 | 100 |
| 40 | 100 | 100 |
| 41 | 100 | 100 |
| 42 | 100 | 100 |
| 43 | 100 | 100 |
| 44 | 100 | 100 |
| 45 | 100 | 100 |
| 46 | 100 | 100 |

| Compound No. | Mortality after 120 hours | |
|---|---|---|
| | 500 ppm | 125 ppm |
| 47 | 100% | 100% |
| 50 | 100 | 100 |
| 52 | 100 | 100 |
| 53 | 100 | 100 |
| 54 | 100 | 100 |
| 55 | 100 | 100 |
| 56 | 100 | 100 |
| 57 | 100 | 100 |
| 58 | 100 | 100 |
| 59 | 100 | 100 |
| 61 | 100 | 100 |
| 62 | 100 | 100 |
| 63 | 100 | 100 |
| 64 | 100 | 100 |
| 65 | 100 | 100 |
| 66 | 100 | 100 |
| 67 | 100 | 100 |
| 68 | 100 | 100 |
| 69 | 100 | 100 |
| 70 | 100 | 100 |
| 72 | 100 | 100 |
| 74 | 100 | 100 |
| 76 | 100 | 100 |
| 78 | 100 | 100 |
| 79 | 100 | 100 |
| 80 | 100 | 100 |
| 81 | 100 | 100 |
| 82 | 100 | 100 |
| 83 | 100 | 100 |
| 84 | 100 | 100 |
| 85 | 100 | 100 |
| 87 | 100 | 100 |

| Compound No. | Mortality after 120 hours | |
|---|---|---|
| | 500 ppm | 125 ppm |
| 88 | 100% | 100% |
| 89 | 100 | 100 |
| 90 | 100 | 100 |
| 91 | 100 | 100 |
| 92 | 100 | 100 |
| 93 | 100 | 100 |
| 95 | 100 | 100 |
| 96 | 100 | 100 |
| 97 | 100 | 100 |
| 102 | 100 | 100 |
| 103 | 100 | 100 |
| 104 | 100 | 100 |
| 105 | 100 | 100 |
| 106 | 100 | 100 |
| 107 | 100 | 100 |
| 109 | 100 | 100 |
| 110 | 100 | 100 |
| 111 | 100 | 100 |
| 112 | 100 | 100 |
| 113 | 100 | 100 |
| 114 | 100 | 100 |
| 115 | 100 | 100 |
| 117 | 100 | 100 |
| 118 | 100 | 100 |
| 119 | 100 | 100 |
| 120 | 100 | 100 |
| 122 | 100 | 100 |
| 123 | 100 | 100 |
| 126 | 100 | 100 |
| 128 | 100 | 100 |
| 129 | 100 | 100 |
| 130 | 100 | 100 |

| Compound No. | Mortality after 120 hours | |
|---|---|---|
| | 500 ppm | 125 ppm |
| 133 | 100% | 100% |
| 134 | 100 | 100 |
| 135 | 100 | 100 |
| 136 | 100 | 100 |
| 137 | 100 | 100 |
| 138 | 100 | 100 |
| 139 | 100 | 100 |
| 144 | 100 | 100 |
| 145 | 100 | 100 |
| 147 | 100 | 100 |
| 148 | 100 | 100 |
| 149 | 100 | 100 |
| 150 | 100 | 100 |
| 151 | 100 | 100 |
| 152 | 100 | 100 |
| 153 | 100 | 100 |
| 154 | 100 | 100 |
| 155 | 100 | 100 |
| 156 | 100 | 100 |
| 157 | 100 | 100 |
| 159 | 100 | 100 |
| 161 | 100 | 100 |
| 162 | 100 | 100 |
| 163 | 100 | 100 |
| 164 | 100 | 100 |
| 165 | 100 | 100 |
| 166 | 100 | 100 |
| 167 | 100 | 100 |
| 168 | 100 | 100 |
| 169 | 100 | 100 |
| 170 | 100 | 100 |
| 171 | 100 | 100 |

| Compound No. | Mortality after 120 hours | |
|---|---|---|
| | 500 ppm | 125 ppm |
| 172 | 100% | 100% |
| 173 | 100 | 100 |
| 174 | 100 | 100 |
| 175 | 100 | 100 |
| 176 | 100 | 100 |
| 177 | 100 | 100 |
| 178 | 100 | 100 |
| 179 | 100 | 100 |
| 180 | 100 | 100 |
| 181 | 100 | 100 |
| 183 | 100 | 100 |
| 184 | 100 | 100 |
| 186 | 100 | 100 |
| 187 | 100 | 100 |
| 190 | 100 | 100 |
| 191 | 100 | 100 |
| 192 | 100 | 100 |
| 193 | 100 | 100 |
| 194 | 100 | 100 |
| 195 | 100 | 100 |
| 196 | 100 | 100 |
| 197 | 100 | 100 |
| 198 | 100 | 100 |
| 199 | 100 | 100 |
| 200 | 100 | 100 |
| 201 | 100 | 100 |
| 202 | 100 | 100 |
| 203 | 100 | 100 |
| 204 | 100 | 100 |
| 205 | 100 | 100 |
| 206 | 100 | 80 |
| 207 | 100 | 100 |

| Compound No. | Mortality after 120 hours | |
| --- | --- | --- |
| | 500 ppm | 125 ppm |
| 208 | 100% | 100% |
| 209 | 100 | 100 |
| 210 | 100 | 100 |
| 211 | 100 | 100 |
| 212 | 100 | 100 |
| 213 | 100 | 100 |
| 214 | 100 | 100 |
| 215 | 100 | 100 |
| 216 | 100 | 100 |
| 217 | 100 | 100 |
| 219 | 100 | 100 |
| 220 | 100 | 100 |
| 221 | 100 | 100 |
| 222 | 100 | 100 |
| 223 | 100 | 100 |
| 224 | 100 | 100 |
| 225 | 100 | 100 |
| Comparative Compound A* | 80 | 20 |

*

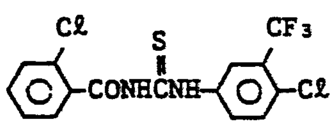

(U.S.P. 3,933,908

Test 2 Insecticidal activity against Tobacco cutworm:

A wettable powder formulated according to the aforesaid Example was diluted with water to the concentrations of 125 ppm and 31.3 ppm of the compound.

A leaf of sweet potato was immersed in the liquid formulation for 30 seconds and air-dried.

The treated leaf was put in a petri dish of 9 cm diameter in which 5 third instar larvae of tobacco cutworm were released and the petri dish was capped with a sheet of glass. The petri dish was placed in a room at 25°C and 65% relative humidity, and the mortality was investigated after 120 hours. The results obtained in two replicates are shown in Table 3.

Table 3

| Compound No. | Mortality after 120 hours | |
|---|---|---|
| | 125 ppm | 31.3 ppm |
| 4 | 100% | 60% |
| 6 | 100 | 60 |
| 10 | 100 | 100 |
| 11 | 100 | 100 |
| 12 | 100 | 60 |
| 14 | 100 | 100 |
| 15 | 100 | 80 |
| 17 | 100 | 100 |
| 18 | 100 | 100 |
| 20 | 100 | 100 |
| 21 | 100 | 100 |
| 22 | 100 | 100 |
| 23 | 100 | 100 |
| 24 | 100 | 100 |
| 25 | 100 | 100 |
| 26 | 100 | 60 |
| 27 | 100 | 100 |
| 30 | 100 | 80 |
| 31 | 100 | 100 |
| 32 | 100 | 100 |
| 33 | 100 | 100 |
| 34 | 100 | 100 |
| 35 | 100 | 100 |
| 36 | 100 | 80 |
| 37 | 100 | 100 |
| 38 | 100 | 80 |
| 39 | 100 | 100 |
| 40 | 100 | 100 |
| 41 | 100 | 100 |
| 42 | 100 | 100 |
| 44 | 100 | 100 |
| 46 | 100 | 100 |

| Compound No. | Mortality after 120 hours 125 ppm | 31.3 ppm |
|---|---|---|
| 47 | 100% | 100% |
| 48 | 100 | 40 |
| 49 | 100 | 100 |
| 50 | 100 | 100 |
| 52 | 100 | 100 |
| 53 | 100 | 100 |
| 54 | 100 | 100 |
| 62 | 100 | 60 |
| 63 | 100 | 100 |
| 64 | 100 | 100 |
| 65 | 100 | 100 |
| 66 | 100 | 70 |
| 67 | 100 | 60 |
| 68 | 100 | 80 |
| 72 | 100 | 100 |
| 76 | 100 | 40 |
| 78 | 100 | 100 |
| 79 | 100 | 100 |
| 80 | 100 | 100 |
| 82 | 100 | 100 |
| 83 | 100 | 100 |
| 84 | 100 | 100 |
| 85 | 100 | 100 |
| 86 | 100 | 100 |
| 87 | 100 | 100 |
| 89 | 100 | 80 |
| 91 | 100 | 100 |
| 92 | 100 | 80 |
| 95 | 100 | 60 |
| 96 | 100 | 100 |
| 97 | 100 | 100 |
| 98 | 100 | 80 |

| Compound No. | Mortality after 120 hours | |
|---|---|---|
| | 125 ppm | 31.3 ppm |
| 100 | 100% | 80% |
| 101 | 100 | 100 |
| 102 | 100 | 100 |
| 103 | 100 | 100 |
| 104 | 100 | 100 |
| 105 | 100 | 100 |
| 106 | 100 | 60 |
| 107 | 100 | 100 |
| 108 | 100 | 100 |
| 109 | 100 | 100 |
| 110 | 100 | 100 |
| 111 | 100 | 100 |
| 112 | 100 | 100 |
| 113 | 100 | 100 |
| 114 | 100 | 100 |
| 115 | 100 | 100 |
| 116 | 100 | 100 |
| 117 | 100 | 100 |
| 118 | 100 | 100 |
| 119 | 100 | 80 |
| 120 | 100 | 100 |
| 121 | 100 | 80 |
| 122 | 100 | 40 |
| 123 | 100 | 40 |
| 124 | 100 | 60 |
| 125 | 100 | 40 |
| 126 | 100 | 100 |
| 127 | 100 | 100 |
| 128 | 100 | 100 |
| 129 | 100 | 100 |
| 130 | 100 | 100 |
| 131 | 100 | 60 |

| Compound No. | Mortality after 120 hours | |
|---|---|---|
| | 125 ppm | 31.3 ppm |
| 133 | 100% | 60% |
| 134 | 100 | 80 |
| 135 | 100 | 100 |
| 136 | 100 | 60 |
| 137 | 100 | 100 |
| 138 | 100 | 60 |
| 141 | 100 | 100 |
| 143 | 100 | 100 |
| 147 | 100 | 100 |
| 148 | 100 | 100 |
| 149 | 100 | 100 |
| 150 | 100 | 100 |
| 151 | 100 | 100 |
| 152 | 100 | 100 |
| 153 | 100 | 100 |
| 154 | 100 | 100 |
| 155 | 100 | 100 |
| 156 | 100 | 100 |
| 157 | 100 | 100 |
| 158 | 100 | 100 |
| 159 | 100 | 100 |
| 160 | 100 | 100 |
| 161 | 100 | 100 |
| 162 | 100 | 100 |
| 163 | 100 | 100 |
| 164 | 100 | 100 |
| 165 | 100 | 100 |
| 166 | 100 | 100 |
| 167 | 100 | 100 |
| 168 | 100 | 100 |
| 169 | 100 | 100 |
| 170 | 100 | 100 |

| Compound No. | Mortality after 120 hours | |
|---|---|---|
| | 125 ppm | 31.3 ppm |
| 172 | 100% | 80% |
| 173 | 100 | 100 |
| 174 | 100 | 100 |
| 175 | 100 | 100 |
| 176 | 100 | 80 |
| 177 | 100 | 80 |
| 178 | 100 | 100 |
| 179 | 100 | 100 |
| 180 | 100 | 80 |
| 181 | 100 | 100 |
| 182 | 100 | 60 |
| 183 | 100 | 80 |
| 184 | 100 | 80 |
| 186 | 100 | 80 |
| 187 | 100 | 60 |
| 188 | 100 | 60 |
| 189 | 100 | 60 |
| 190 | 100 | 80 |
| 191 | 100 | 100 |
| 192 | 100 | 100 |
| 193 | 100 | 100 |
| 194 | 100 | 100 |
| 195 | 100 | 100 |
| 196 | 100 | 100 |
| 198 | 100 | 100 |
| 199 | 100 | 100 |
| 200 | 100 | 100 |
| 201 | 100 | 100 |
| 202 | 100 | 100 |
| 203 | 100 | 100 |
| 204 | 100 | 100 |
| 205 | 100 | 100 |

| Compound No. | Mortality after 120 hours | |
|---|---|---|
| | 125 ppm | 31.3 ppm |
| 207 | 100% | 100% |
| 208 | 100 | 80 |
| 209 | 100 | 100 |
| 210 | 100 | 100 |
| 211 | 100 | 100 |
| 212 | 100 | 100 |
| 213 | 100 | 100 |
| 214 | 100 | 100 |
| 215 | 100 | 100 |
| 216 | 100 | 100 |
| 217 | 100 | 100 |
| 219 | 100 | 100 |
| 220 | 100 | 100 |
| 221 | 100 | 100 |
| 222 | 100 | 100 |
| 223 | 100 | 100 |
| 224 | 100 | 100 |
| 225 | 100 | 100 |
| 226 | 80 | 60 |
| 227 | 100 | 100 |
| Comparative Compound B* | 80 | 10 |

*

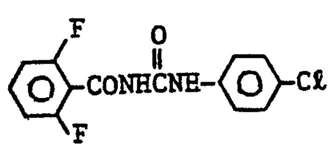

(diflubenzuron)

Test 3 Insecticidal activity against Diamondback moth:

An emulsifiable concentrate or a wettable powder (in case of metal salt of the compound) was diluted with water to the concentrations of 500 ppm and 125 ppm of the compound.

A leaf of cabbage was immersed in the liquid formulation for 30 seconds and air-dried.

The treated leaf was put in a petri dish of 9 cm diameter in which 5 third instar larvae of diamondback moth were released, and the petri dish was capped with a sheet of glass. The petri dish was kept in a room at 25°C and 65% relative humidity, and the mortality was investigated after 120 hours. The results obtained in two replicates are shown in Table 4.

Table 4

| Compound | Mortality after 120 hours | |
|---|---|---|
| No. | 500 ppm | 125 ppm |
| 2 | 80% | 40% |
| 4 | 100 | 100 |
| 6 | 100 | 100 |
| 7 | 100 | 100 |
| 10 | 100 | 100 |
| 11 | 100 | 100 |
| 14 | 100 | 100 |
| 17 | 100 | 100 |
| 21 | 100 | 100 |
| 25 | 100 | 100 |
| 27 | 100 | 100 |
| 29 | 100 | 100 |
| 30 | 100 | 100 |
| 31 | 100 | 100 |
| 35 | 100 | 100 |
| 38 | 100 | 100 |
| 42 | 100 | 100 |
| 44 | 100 | 100 |
| 45 | 100 | 100 |
| 47 | 100 | 100 |
| 50 | 100 | 100 |
| 51 | 100 | 100 |
| 54 | 100 | 100 |
| 61 | 100 | 60 |
| 62 | 100 | 90 |
| 63 | 100 | 100 |
| 64 | 100 | 100 |
| 65 | 100 | 100 |
| 66 | 100 | 100 |
| 72 | 100 | 100 |
| 73 | 100 | 80 |
| 76 | 100 | 100 |

| Compound No. | Mortality after 120 hours | |
|---|---|---|
| | 500 ppm | 125 ppm |
| 78 | 100% | 100% |
| 79 | 100 | 100 |
| 80 | 100 | 100 |
| 82 | 100 | 100 |
| 83 | 100 | 100 |
| 84 | 100 | 100 |
| 85 | 100 | 100 |
| 86 | 100 | 100 |
| 87 | 100 | 100 |
| 89 | 100 | 100 |
| 91 | 100 | 100 |
| 92 | 100 | 100 |
| 93 | 100 | 100 |
| 95 | 100 | 100 |
| 96 | 100 | 100 |
| 97 | 100 | 100 |
| 100 | 100 | 100 |
| 101 | 100 | 100 |
| 102 | 100 | 100 |
| 103 | 100 | 100 |
| 104 | 100 | 100 |
| 105 | 100 | 100 |
| 106 | 100 | 100 |
| 107 | 100 | 100 |
| 109 | 100 | 100 |
| 110 | 100 | 100 |
| 111 | 100 | 100 |
| 112 | 100 | 100 |
| 113 | 100 | 100 |
| 114 | 100 | 100 |
| 115 | 100 | 100 |
| 117 | 100 | 100 |

| Compound No. | Mortality after 120 hours 500 ppm | 125 ppm |
|---|---|---|
| 118 | 100% | 100% |
| 192 | 100 | 100 |
| 193 | 100 | 100 |
| 194 | 100 | 100 |
| 195 | 100 | 100 |
| 196 | 100 | 100 |
| 197 | 100 | 100 |
| 199 | 100 | 100 |
| 200 | 100 | 100 |
| 201 | 100 | 100 |
| 202 | 100 | 100 |
| 203 | 100 | 100 |
| 204 | 100 | 100 |
| 205 | 100 | 100 |
| 206 | 100 | 80 |
| 207 | 100 | 100 |
| 209 | 100 | 100 |
| 210 | 100 | 100 |
| 211 | 100 | 100 |
| 212 | 100 | 100 |
| 213 | 100 | 100 |
| 214 | 100 | 100 |
| 215 | 100 | 100 |
| 216 | 100 | 100 |
| 217 | 100 | 100 |
| 218 | 100 | 80 |
| 219 | 100 | 100 |
| 220 | 100 | 100 |
| 221 | 100 | 100 |
| 222 | 100 | 100 |
| 223 | 100 | 100 |
| Comparative Compound B* | 60 | 0 |

* The same with Test 2

<u>Test 4</u> Ovicidal activity against eggs of Tobacco cutworm:

An emulsifiable concentrate or a wettable powder (in case of metal salt of the compound) was diluted with water to the concentrations of 500 ppm and 125 ppm of the compound.

Eggs of tobacco cutworm were immersed in the liquid formulation for 30 seconds and air-dried.

The eggs were put in a petri dish and the petri dish was capped with a sheet of glass. The petri dish was kept in a room at 25°C and 65% relative humidity, and the ovicidal activity was investigated after 7 days. The results are shown in Table 5.

Table 5

| Compound No. | Ovicidal activity after 7 days | |
|---|---|---|
| | 500 ppm | 125 ppm |
| 1 | 100% | 100% |
| 2 | 100 | 80 |
| 3 | 100 | 100 |
| 4 | 100 | 100 |
| 5 | 100 | 100 |
| 6 | 100 | 100 |
| 7 | 82 | 73 |
| 8 | 100 | 100 |
| 9 | 95 | 81 |
| 10 | 100 | 94 |
| 11 | 100 | 100 |
| 12 | 100 | 100 |
| 13 | 100 | 92 |
| 14 | 100 | 100 |
| 15 | 100 | 100 |
| 16 | 100 | 95 |
| 17 | 80 | 65 |
| 18 | 100 | 100 |
| 19 | 100 | 100 |
| 21 | 100 | 100 |
| 22 | 100 | 100 |
| 23 | 100 | 96 |
| 24 | 100 | 100 |
| 26 | 100 | 95 |
| 27 | 100 | 100 |
| 28 | 90 | 56 |
| 29 | 100 | 100 |
| 30 | 98 | 95 |
| 31 | 96 | 94 |
| 32 | 100 | 100 |
| 33 | 100 | 100 |
| 34 | 96 | 91 |

| Compound No. | Ovicidal activity after 7 days | |
|---|---|---|
| | 500 ppm | 125 ppm |
| 35 | 98% | 95% |
| 36 | 100 | 82 |
| 37 | 100 | 100 |
| 38 | 100 | 100 |
| 39 | 100 | 97 |
| 40 | 100 | 100 |
| 41 | 100 | 100 |
| 42 | 98 | 93 |
| 43 | 100 | 100 |
| 55 | 100 | 100 |
| 60 | 100 | 79 |
| 61 | 100 | 100 |
| 62 | 97 | 94 |
| 63 | 100 | 100 |
| 64 | 100 | 100 |
| 65 | 100 | 100 |
| 66 | 100 | 100 |
| 67 | 100 | 100 |
| 68 | 100 | 100 |
| 69 | 100 | 100 |
| 70 | 100 | 100 |
| 71 | 100 | 100 |
| 74 | 100 | 100 |
| 77 | 100 | 100 |
| 78 | 100 | 100 |
| 79 | 100 | 100 |
| 81 | 100 | 100 |
| 82 | 100 | 100 |
| 84 | 100 | 100 |
| 85 | 100 | 100 |
| 86 | 100 | 100 |
| 87 | 100 | 100 |

| Compound No. | Ovicidal activity after 7 days | |
|---|---|---|
| | 500 ppm | 125 ppm |
| 88 | 100% | 100% |
| 89 | 100 | 100 |
| 90 | 100 | 100 |
| 91 | 100 | 100 |
| 92 | 100 | 100 |
| 93 | 100 | 100 |
| 94 | 100 | 100 |
| 95 | 100 | 100 |
| 96 | 100 | 100 |
| 98 | 100 | 100 |
| 99 | 100 | 100 |
| 100 | 100 | 100 |
| 101 | 100 | 100 |
| 102 | 100 | 100 |
| 103 | 100 | 100 |
| 104 | 100 | 100 |
| 105 | 100 | 100 |
| 106 | 100 | 100 |
| 107 | 100 | 94 |
| 109 | 100 | 100 |
| 110 | 100 | 100 |
| 111 | 100 | 100 |
| 112 | 100 | 100 |
| 113 | 100 | 100 |
| 114 | 100 | 100 |
| 115 | 100 | 100 |
| 116 | 100 | 100 |
| 117 | 100 | 100 |
| 119 | 100 | 100 |
| 120 | 100 | 100 |
| 121 | 100 | 100 |
| 122 | 100 | 100 |

| Compound No. | Ovicidal activity after 7 days | |
|---|---|---|
| | 500 ppm | 125 ppm |
| 126 | 100% | 100% |
| 134 | 100 | 90 |
| 141 | 100 | 100 |
| 147 | 100 | 100 |
| 148 | 100 | 100 |
| 149 | 100 | 100 |
| 150 | 100 | 100 |
| 151 | 100 | 100 |
| 152 | 100 | 100 |
| 153 | 100 | 100 |
| 154 | 100 | 100 |
| 155 | 100 | 100 |
| 156 | 100 | 100 |
| 157 | 100 | 100 |
| 158 | 100 | 100 |
| 159 | 100 | 100 |
| 160 | 100 | 100 |
| 161 | 100 | 100 |
| 162 | 100 | 100 |
| 163 | 100 | 100 |
| 164 | 100 | 100 |
| 165 | 100 | 100 |
| 166 | 100 | 100 |
| 167 | 100 | 100 |
| 168 | 100 | 100 |
| 169 | 100 | 90 |
| 170 | 100 | 95 |
| 171 | 100 | 100 |
| 172 | 100 | 100 |
| 173 | 100 | 100 |
| 174 | 100 | 97 |
| 175 | 100 | 100 |
| 176 | 100 | 100 |

| Compound No. | Ovicidal activity after 7 days | |
|---|---|---|
| | 500 ppm | 125 ppm |
| 177 | 100% | 100% |
| 178 | 100 | 100 |
| 179 | 100 | 100 |
| 180 | 100 | 97 |
| 181 | 100 | 100 |
| 182 | 100 | 87 |
| 183 | 100 | 100 |
| 184 | 100 | 100 |
| 186 | 100 | 100 |
| 188 | 100 | 93 |
| 190 | 100 | 97 |
| 191 | 100 | 100 |
| 192 | 100 | 100 |
| 193 | 100 | 100 |
| 194 | 100 | 100 |
| 195 | 100 | 100 |
| 196 | 100 | 100 |
| 198 | 100 | 100 |
| 200 | 100 | 100 |
| 201 | 100 | 94 |
| 203 | 100 | 100 |
| 204 | 100 | 100 |
| 207 | 100 | 100 |
| 210 | 100 | 97 |
| 212 | 100 | 98 |
| 213 | 100 | 92 |
| 214 | 100 | 100 |
| 215 | 100 | 100 |
| 216 | 100 | 100 |
| 219 | 100 | 100 |
| 223 | 100 | 98 |
| Comparative Compound A* | 48 | 13 |

* The same with Test 1.

What we claim is:

1. A compound having the formula

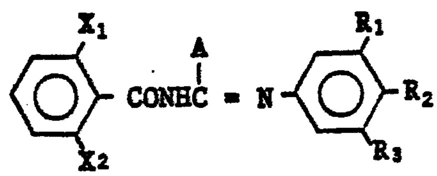

wherein each of $X_1$ and $X_2$ is hydrogen, halogen, methyl, trifluormethyl, with the proviso that $X_1$ and $X_2$ can not be hydrogen at the same time, and

each of $R_1$ and $R_3$ is hydrogen, halogen, nitro, $C_{1-6}$ haloalkyl, $C_{2-6}$ alkenyloxy, or aryloxy radical selected from a group consisting of phenoxy and quinoxalinyloxy which may be substituted by nitro and/or $C_{1-6}$ haloalkyl on said aromatic ring; and

$R_2$ is hydrogen, halogen, nitro, $C_{1-8}$ alkyl, $C_{1-3}$ haloalkyl, $C_{1-6}$ alkoxycarbonyl, dialkylamino, phenylazo which may be substituted by $C_{1-3}$ alkyl, or $Y-R_4$, wherein Y is O or S and $R_4$ is $C_{1-6}$ alkyl, $C_{1-6}$ haloalkyl, $C_{2-6}$ alkenyl, $C_{2-6}$ haloalkenyl, $C_{2-6}$ alkynyl, phenylalkyl which may be substituted by halogen and/or $C_{1-3}$ haloalkyl, aromatic radical selected from a group consisting of phenyl, pyridyl and quinoxalinyl which may be substituted by halogen, nitro, cyano and/or $C_{1-3}$ haloalkyl on said aromatic ring; and

A is Az or $-SS-R_5$, wherein Az is heterocyclic radical selected from a group consisting of 1-benzimidazolyl, 1-benztriazolyl and 1-pyrazolyl, which may be substituted by $C_{1-3}$ alkyl and/or halogen, or heterocyclic radical selected from a group consisting of 1-triazolyl and 1-imidazolyl, which may be substituted by $C_{1-3}$ alkyl, and $R_5$ is $C_{1-18}$ alkyl,

$C_{3-8}$ cycloalkyl, phenylalkyl of phenyl which may be substituted by halogen, nitro and/or $C_{1-8}$ alkyl; or its metal salt.

2. A compound according to claim 1, wherein A is heterocyclic radical selected from a group consisting of 1-benzimidazolyl, 1-benztriazolyl and 1-pyrazolyl, which may be substituted by $C_{1-3}$ alkyl and/or halogen, or heterocyclic radical selected from a group consisting of 1-triazolyl and 1-imidazolyl, which may be substituted by $C_{1-3}$ alkyl.

3. A compound according tot claim 1, wherein A is $-SS-R_5$, in which $R_5$ is $C_{1-18}$ alkyl, $C_{3-8}$ cycloalkyl, phenylalkyl of phenyl which may be substituted by halogen, nitro and/or $C_{1-8}$ alkyl.

4. An insecticidal composition comprising an inert carrier and an effective amount of a compound of claim 1.

5. An insecticidal composition comprising an inert carrier and an effective amount of a compound of claim 2.

6. An insecticidal composition comprising an inert carrier and an effective amount of a compound of claim 3.

7. A process for the controlling insects comprising applying to the locus of plants an effective amount of a compound of claim 1.

8. A process for the controlling insects comprising applying to the locus of plants an effective amount of a compound of claim 2.

9. A process for the controlling insects comprising applying to the locus of plants an effective amount of a compound of claim 3.

10. A process for the preparation of a compound having the formula

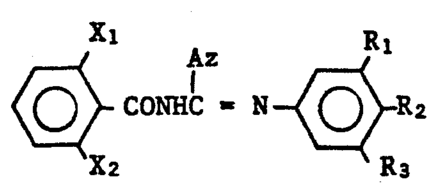

which comprises reacting a compound having the formula

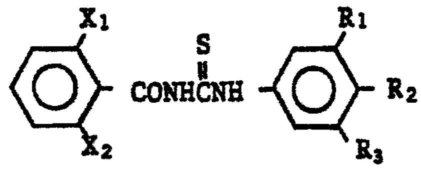

with a compound having the formula Az-SO-Az

wherein each of $X_1$ and $X_2$ is hydrogen, halogen, methyl, trifluormethyl, with the proviso that $X_1$ and $X_2$ can not be hydrogen at the same time, and

each of $R_1$ and $R_3$ is hydrogen, halogen, nitro, $C_{1-6}$ haloalkyl, $C_{2-6}$ alkenyloxy, or aryloxy radical selected from a group consisting of phenoxy and quinoxalinyloxy which may be substituted by nitro and/or $C_{1-6}$ haloalkyl on said aromatic ring; and

$R_2$ is hydrogen, halogen, nitro, $C_{1-8}$ alkyl, $C_{1-3}$ haloalkyl, $C_{1-6}$ alkoxycarbonyl, dialkylamino, phenylazo which may be substituted by $C_{1-3}$ alkyl, or Y-$R_4$, wherein Y is O or S and $R_4$ is $C_{1-6}$ alkyl, $C_{1-6}$ haloalkyl, $C_{2-6}$ alkenyl, $C_{2-6}$ haloalkenyl, $C_{2-6}$ alkynyl, phenylalkyl which may be substituted by halogen and/or $C_{1-3}$ haloalkyl, aromatic radical selected from a group consisting of phenyl, pyridyl and quinoxalinyl which may be substituted by halogen, nitro, cyano and/or $C_{1-3}$ haloalkyl on said aromatic ring; and

Az is heterocyclic radical selected from a group consisting of 1-benzimidazolyl, 1-benztriazolyl and 1-pyrazolyl, which may be substituted by $C_{1-3}$ alkyl and/or halogen, or heterocyclic radical selected from a group consisting of 1-triazolyl and 1-imidazolyl, which may be substituted by $C_{1-3}$ alkyl.

11. A process for the preparation of a compound having the formula

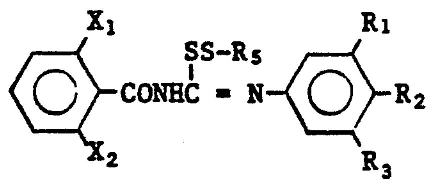

which comprises reacting a compound having the formula

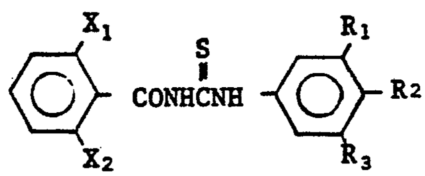

with a compound having the formula $R_5S$-Hal

wherein each of $X_1$ and $X_2$ is hydrogen, halogen, methyl, trifluormethyl, with the proviso that $X_1$ and $X_2$ can not be hydrogen at the same time, and

each of $R_1$ and $R_3$ is hydrogen, halogen, nitro, $C_{1-6}$ haloalkyl, $C_{2-6}$ alkenyloxy, or aryloxy radical selected from a group consisting of phenoxy and quinoxalinyloxy which may be substituted by nitro and/or $C_{1-6}$ haloalkyl on said aromatic ring; and

$R_2$ is hydrogen, halogen, nitro, $C_{1-8}$ alkyl, $C_{1-3}$ haloalkyl, $C_{1-6}$ alkoxycarbonyl, dialkylamino, phenylazo which may be substituted by $C_{1-3}$ alkyl, or Y-$R_4$, wherein Y is O or S and $R_4$ is $C_{1-6}$ alkyl, $C_{1-6}$ haloalkyl, $C_{2-6}$ alkenyl, $C_{2-6}$ haloalkenyl, $C_{2-6}$ alkynyl, phenylalkyl which may be substituted by halogen and/or $C_{1-3}$ haloalkyl, aromatic radical selected from a group consisting

of phenyl, pyridyl and quinoxalinyl which may be substituted by halogen, nitro, cyano and/or $C_{1-3}$ haloalkyl on said aromatic ring; and

$R_5$ is $C_{1-18}$ alkyl, $C_{3-8}$ cycloalkyl, phenylalkyl of phenyl which may be substituted by halogen, nitro and/or $C_{1-8}$ alkyl; and Hal is halogen.

12. A process for preparation of a compound having the formula

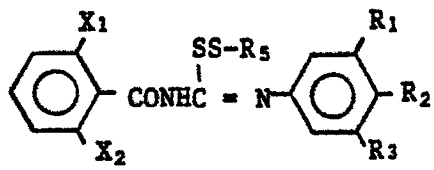

which comprises reacting a compound having the formula

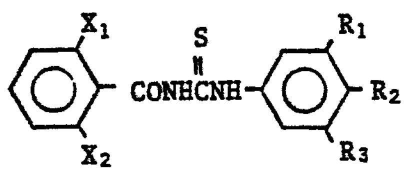

with a compound having the formula

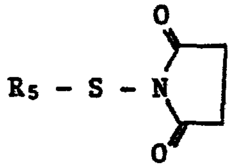

wherein each of $X_1$ and $X_2$ is hydrogen, halogen, methyl, trifluormethyl, with the proviso that $X_1$ and $X_2$ can not be hydrogen at the same time, and

each of $R_1$ and $R_3$ is hydrogen, halogen, nitro, $C_{1-6}$ haloalkyl, $C_{2-6}$ alkenyloxy, or aryloxy radical selected from a group consisting of phenoxy and quinoxalinyloxy which may be substituted by nitro and/or $C_{1-6}$ haloalkyl on said aromatic ring; and

$R_2$ is hydrogen, halogen, nitro, $C_{1-8}$ alkyl, $C_{1-3}$ haloalkyl, $C_{1-6}$ alkoxycarbonyl, dialkylamino, phenylazo which may be substituted by $C_{1-3}$ alkyl, or Y-$R_4$, wherein Y is O or S and $R_4$ is $C_{1-6}$ alkyl, $C_{1-6}$ haloalkyl, $C_{2-6}$ alkenyl, $C_{2-6}$ haloalkenyl, $C_{2-6}$ alkynyl, phenylalkyl which may be substituted by halogen and/or $C_{1-3}$ haloalkyl, aromatic radical selected from a group consisting of phenyl, pyridyl and quinoxalinyl which may be substituted by halogen, nitro, cyano and/or $C_{1-3}$ haloalkyl on said aromatic ring; and

$R_5$ is $C_{1-18}$ alkyl, $C_{3-8}$ cycloalkyl, phenylalkyl of phenyl which may be substituted by halogen, nitro and/or $C_{1-8}$ alkyl.